(19) **Europäisches Patentamt**

**European Patent Office**

**Office européen des brevets**

(11) **EP 0 743 898 B1**

(12) **EUROPÄISCHE PATENTSCHRIFT**

(45) Veröffentlichungstag und Bekanntmachung des Hinweises auf die Patenterteilung:
**02.11.2000 Patentblatt 2000/44**

(21) Anmeldenummer: **95907649.8**

(22) Anmeldetag: **06.02.1995**

(51) Int. Cl.$^7$: **B29C 67/20**

(86) Internationale Anmeldenummer:
**PCT/EP95/00421**

(87) Internationale Veröffentlichungsnummer:
**WO 95/21053 (10.08.1995 Gazette 1995/34)**

(54) **VERFAHREN ZUR HERSTELLUNG EINES WERKSTOFFES**

PROCESS FOR PRODUCING A MATERIAL

PROCEDE DE PRODUCTION D'UN MATERIAU

(84) Benannte Vertragsstaaten:
**AT BE CH DE DK ES FR GB IT LI LU NL SE**

(30) Priorität: **04.02.1994 DE 4403509**

(43) Veröffentlichungstag der Anmeldung:
**27.11.1996 Patentblatt 1996/48**

(73) Patentinhaber:
**Draenert, Klaus, Dr.med.Dr.med.habil.
D-81545 München (DE)**

(72) Erfinder:
**Draenert, Klaus, Dr.med.Dr.med.habil.
D-81545 München (DE)**

(56) Entgegenhaltungen:
**DE-A- 2 242 867          DE-A- 4 033 291
GB-A- 1 139 317**

- **PATENT ABSTRACTS OF JAPAN vol. 013 no. 431
(C-640) ,26.September 1989 & JP,A,01 165732
(KAWASAKI STEEL CORP) 29.Juni 1989,**

## Beschreibung

Die Erfindung betrifft ein Verfahren zur Herstellung eines Werkstoffs mit einem Gerüst aus schalenartigen Strukturen und einem interkonnektierenden Porensystem, ein Verfahren zur Herstellung eines Werkstoffs aus miteinander über Stege verbundenen Formkörpern, sowie ein Verfahren zur Herstellung eines Werkstoffs mit einem Gerüst aus schalenartigen Strukturen, dessen interkonnektierendes Porensystem mit über Stege verbundenen Formkörpern gefüllt ist.

Verlorener Knochen, beispielsweise als Folge von Unfalltraumen oder nach Resektion von Tumoren, nach Infektionen oder durch idiopathisch sich entwickelnde Knochenzysten, stellt seit langem ein großes Problem für die Chirurgen dar. So wurde beispielsweise versucht, Knochen künstlich zu ersetzen, Tierknochen zu verwenden, Tierknochen entsprechend so aufzubereiten, daß er beim Menschen nach seinem Einsatz nicht abgestoßen wird oder auch Knochen von Menschen in Gefriertruhen zu konservieren und als gefrorenen homologen Knochen einzusetzen.

Für die Aufbereitung von Tierknochen für die Verwendung beim Menschen gibt es vielfältige Methoden. Beispielsweise wurde auch Elfenbein verwendet. Ein Knochenersatz, der besonders weit verbreitet Eingang in die Chirurgie gefunden hat, ist der sogenannte "Kieler Knochenspan".

Alle chemisch aufbereiteten heterologen Transplantate vom Tier werden jedoch unvollständig oder gar nicht in den Wirtsknochen eingebaut. Der Bankknochen führt sehr oft zu Infektionen, u.a. zu Aids-Infektionen, mit tödlichem Ausgang. Frische homologe Knochentransplantate sind in noch weit höherem Maße von derartigen Komplikationen betroffen. Hierbei können sogar Tumoren transplantiert werden. Es wurde daher immer wieder versucht, Knochenersatzwerkstoffe und Methoden zu entwickeln, durch die die Nachteile der Immunreaktion und der Übertragung von Krankheiten vermieden werden können.

In der DE-A-39 03 695 wird ein Verfahren zur Herstellung von als Knochenersatzmaterial verwendbarer resorbierbarer Keramik auf der Basis von Tricalciumphosphat beschrieben, bei dem natürliches, von Weichteilen befreites Knochenmaterial als Ausgangsmaterial verwendet wird. Aus dem Knochenmaterial wird durch Pyrolyse restliche organische Substanz entfernt, und das verbleibende, aus Hydroxylapatit bestehende Knochenmaterial wird anschließend mit Phosphatträgern behandelt, und dann einer Sinterung unterzogen. Dieses bovine geglühte Knochenmaterial zeigt auch gute Einheilungseigenschaften.

Die genannten Werkstoffe tierischen Ursprungs können jedoch die an Knochenersatzmaterial in bezug auf die Reproduzierbarkeit der Struktur gestellten Forderungen, insbesondere im Hinblick auf die Materialfestigkeit und auf die Standardisierbarkeit des Herstellungsverfahrens, nicht erfüllen und sind außerdem sehr teuer in der Herstellung. Es ist deshalb in der Medizin wünschenswert, die verwendeten Werkstoffe, beispielsweise Knochenersatzwerkstoffe, voll synthetisch herzustellen. Diese Werkstoffe sollten bei einem Minimum an Material ein Höchstmaß an Festigkeit garantieren und die verschiedensten Eigenschaften für die verschiedensten Indikationen zur Verfügung stellen. Beispielsweise ist es wünschenswert, ein Verfahren bereitzustellen, mit dem einerseits knochenähnliche trabekuläre Strukturen, d.h. ein "Positiv" des Knochens, und andererseits ein "Negativ" des Knochens, d.h. formgebende Negativformen der Markräume des Knochens, herstellbar sind. Durch ein derartiges Verfahren könnte einerseits Knochen in seiner physiologischen Struktur nachgebildet und es könnten andererseits, durch das Negativ, Stütz- und Leitgerüste vorgegeben werden, die vom Knochen umwachsen werden und sehr rasch in der Lage sind, hohe Belastungen aufzunehmen.

In der DE-A-40 33 291 ist ein Verfahren beschrieben, mit dem sich die genannten Forderungen in mancher Beziehung erreichen lassen. Bei diesem Verfahren werden zunächst vorzugsweise kugelförmige Formkörper zu einem dreidimensionalen Formkörper-Konglomerat miteinander verbunden, danach wird ein sich vom Material der Formkörper unterscheidendes Material um die Formkörper herum geformt, um ein dreidimensionales Gerüst auszubilden, und anschließend werden die Formkörper entfernt, so daß lediglich das dreidimensionale Gerüst verbleibt. In anschließenden Verfahrensschritten kann das durch das Entfernen der Formkörper entstandene Hohlraumsystem erneut beispielsweise mit einer Keramikmasse gefüllt und anschließend das dreidimensionale Gerüst entfernt werden. Mit diesem Verfahren läßt sich somit sowohl ein "Positiv" des Knochens als auch das vorstehend beschriebene "Negativ" herstellen. Die Herstellung des als Ausgangsmaterial dienenden Formkörper-Konglomerats (Negativ) ist jedoch recht aufwendig und nicht in jedem Falle reproduzierbar.

Aus der DE-A-22 42 867 ist ein Verfahren zur Herstellung implantierbarer, poröser, keramischer Knochenersatz-, Knochenverbund- oder Prothesenverankerungswerkstoffe bekannt. Bei diesem Verfahren wird zunächst ein in etwa den Poren und Porenverbindungen des fertigen Werkstoffes entsprechendes "Hilfsgerüst" aus Kugeln hergestellt, das danach mit einer gießfähigen keramischen Masse ausgegossen wird. Nach dem zumindest teilweisen Aushärten der keramischen Masse wird schließlich das Hilfsgerüst abgebaut und entfernt. Das zur Herstellung des anfänglichen Hilfsgerüsts aus Kugeln beschriebene Verfahren ist jedoch allenfalls unter großem Aufwand zu standardisieren und kaum reproduzierbar.

Eine Aufgabe der Erfindung besteht somit darin, Verfahren bereitzustellen, die es ermöglichen, einerseits einen Werkstoff mit einem dreidimensionalen Gerüst aus schalenartigen oder trabekulären Strukturen als Positiv und andererseits auch dessen Negativ, d.h. einen Werkstoff aus miteinander verbundenen Formkörpern, sowie eine Kombination aus diesen beiden Werkstoffen bereitzustellen, wobei sowohl die Porosität und die Dicke der tragenden Strukturen des Positiv-Werkstoffes als auch die Konfiguration der Formkörper und deren Verbindungen im Negativ-Werkstoff unter Berücksichtigung der jeweils geforderten Eigenschaften, wie Formstabilität und Resorbierbarkeit, nach den jeweiligen vorgegebenen Bedürfnissen exakt und reproduzierbar einstellbar sind.

Eine weitere Aufgabe der Erfindung besteht darin, Verfahren zur Herstellung derartiger Werkstoffe bereitzustellen, welche erhöhte Festigkeit und/oder Durchlässigkeit aufweisen.

Diese Aufgaben werden durch die vorliegende Erfindung gelöst. Die Erfindung löst somit ein altes Problem in der Werkstoffherstellung, insbesondere in der Herstellung von Werkstoffen für die Medizin. Mit dem erfindungsgemäßen Verfahren läßt sich ein poröser Werkstoff mit einem durchgehend porösen Gerüst mit einer einstellbaren Porosität und einer einstellbaren Festigkeit sowie jeweils den Bedürfnissen angepaßten Materialeigenschaften, wie Löslichkeit und Resorbierbarkeit, einfach und reproduzierbar herstellen, wobei das Porensystem gegebenenfalls mit einem unterschiedlichen Material gefüllt sein kann.

Der fertige Werkstoff als "Positiv", d.h. als dem Knochen nachgebildeter Werkstoff, weist ein dreidimensionales Gerüst aus tragenden, schalenartigen Strukturen auf, die miteinander in Verbindung stehende und vorgebbar einstellbare Hohlräume umschließen. Diese Hohlräume sind insbesondere dadurch vorgebbar einstellbar, daß der im fertigen Werkstoff durch die Hohlräume eingenommene Raum zunächst während des Herstellungsverfahrens des Werkstoffs durch Formkörper als Platzhalter eingenommen wird, wobei die Formkörper je nach den Anforderungen an das Porensystem in geeigneter Weise ausgewählt und in einer Form angeordnet werden. Der fertige Werkstoff als "Negativ", der beispielsweise als Implantat verwendbar ist, weist einen dreidimensionalen Verbund aus Formkörpern auf, die über einstellbar breite und lange Stege miteinander verbunden sind. Die Form und Ausbildung dieser "Stege" wird nachstehend näher erläutert. Der Werkstoff als Positiv ist dadurch herstellbar, daß das Gerüst um als Platzbalter für die Hohlräume dienende deformierbare Formkörper, die ein "Negativ" bilden, ausgebildet wird und die Formkörper anschließend entfernt werden.

Der fertige Werkstoff als Negativ ist durch Ausgießen des Gerüsts, d.h. des Positivwerkstoffes, und anschließendes Entfernen des Gerüsts herstellbar.

Je nach Materialwahl ist es auch möglich, um diesen fertigen Negativwerkstoff erneut ein Gerüst auszubilden, Z.B. durch Ausgießen mit einem gießfähigen Material, und nach dem Verfestigen des gießfähigen Materials das Negativ zu entfernen. Der auf diese Weise hergestellte Positivwerkstoff entspricht in seiner äußeren Form dem Gerüst des vorstehend beschriebenen Positivwerkstoffs, kann jedoch aus einem anderen Material bestehen.

Durch Kombination verschiedener Materialien sind somit sowohl Positiv- als auch Negativ-Werkstoffe aus den verschiedensten resorbierbaren und nicht-resorbierbaren Materialien herstellbar. Der Werkstoff kann beispielsweise als Kunstknochen, als Implantat, als Filter und als drug delivery system verwendet werden.

Die Verwendung des Werkstoffes in der Filtertechnologie ist deshalb besonders vorteilhaft, da jede beliebige Porosität einstellbar ist und außerdem ein makroskopisch großporiges Filtersystem mit einem mikroporösen Filtersystem kombiniert werden kann, wenn der Grundwerkstoff selbst porös ist. Makro- und Mikroporosität können außerdem teilweise oder vollständig mit einem aktiv absorbierenden Material gefüllt werden.

Für bestimmte Anwendungen kann es auch wünschenswert sein, einen Werkstoff bereitzustellen, bei dem Positiv und Negativ aus unterschiedlichen Materialien gemeinsam vorliegen, beispielsweise einen Werkstoff mit einem Metallgerüst aus schalenartigen Strukturen, dessen interkonnektierendes Porensystem, d.h. das "Negativ", mit einer keramischen Masse, wie Tricalciumphosphat oder Hydroxylapatit, gefüllt ist. Bei Verwendung eines derartigen Werkstoffs, beispielsweise als Implantat, wächst der Knochen zunächst gut an die Keramik an, und es kann sich ein Primärverbund zwischen Knochen und Keramik ausbilden. Das weitere Einwachsen des Knochens wird durch die osteokonduktive Wirkung der Keramik ebenfalls begünstigt. Vorzugsweise wird hierbei eine resorbierbare Keramik verwendet, die Keramik kann jedoch auch nicht resorbierbar sein. Der Werkstoff aus Positiv und Negativ kann gegebenenfalls auch eine höhere Festigkeit als der reine Positiv-Werkstoff aufweisen.

Als Ausgangsmaterial für das erfindungsgemäße Verfahren werden deformierbare Formkörper verwendet. Für die Formkörper bevorzugt ist ein Material, das einen relativ niedrigen Elastizitätsmodul aufweist und unter Druck relativ leicht verformbar ist. Die Formkörper, die als Platzhalter für das Hohlraumsystem des fertigen porösen Werkstoffes dienen, weisen vorzugsweise die Form von Kugeln auf, können aber auch Ellipsoide, Granulatkörper oder Vielecke sein oder aus Mischungen derartiger Formkörper bestehen. Das Material für die Formkörper wird derart gewählt, daß die Formkörper sich später während des weiteren Verfahrens leicht wieder herauslösen lassen, beispielsweise auf chemische oder physikalische Weise.

Die Formkörper werden zunächst in eine Form gefüllt und gegebenenfalls gerüttelt und bilden eine Schüttung. Hierbei werden die Formkörper vorzugsweise noch nicht wesentlich deformiert. Es ist auch nicht erforderlich, daß die Formkörper chemisch oder physikalisch miteinander verbunden werden. Vielmehr genügt es, daß die Formkörper bei Befüllen der Form mit einem gieß-, spritz- oder schüttfähigen Material ausreichend aneinandergedrückt werden, was beispielsweise durch geeignete Einstellung des Einspritzdrucks des Materials oder durch Druckerzeugung auf andere Weise leicht steuerbar ist. Durch die Druckbeaufschlagung werden die Formkörper leicht aneinandergepreßt und/oder deformiert. Durch die Stärke der Druckbeaufschlagung läßt sich der Grad der Verformung der Formkörper und damit auch die Breite bzw. die Querschnittsfläche von deren Kontaktstellen und deren Gesamtkonfiguration, die später im fertigen porösen Werkstoff dem interkonnektierenden Porensystem entspricht, leicht und präzise einstellen und steuern. Zwar ist auch in der DE-A-40 33 291 die Einstellbarkeit des dreidimensionalen Gerüsts aus bälkchenartigen Strukturen beschrieben; mit dem erfindungsgemäßen Verfahren läßt sich aber beim Negativmodell die Breite bzw. der Querschnitt der Stege, durch die die Formkörper miteinander verbunden sind, viel feiner und reproduzierbarer einstellen, da der Auflagedruck zwischen den einzelnen Formkörpern nicht von Luft und anderen Fehleinschlüssen beeinflußt wird, wie es der Fall ist, wenn die einzelnen Formkörper miteinander chemisch oder physikalisch zu einem Formkörperkonglomerat verbunden werden. Wenn in diesem Zusammenhang der Begriff "Stege" verwendet wird, ist damit generell die Verbindung zwischen zwei benachbarten und miteinander in Berührung stehenden Formkörpern gemeint. Je stärker die Formkörper deformiert und gegeneinander gedrückt werden, um so größer ist unter ansonsten unveränderten Voraussetzungen der Querschnitt der Verbindung bzw. des "Stegs" zwischen den beiden Formkörpern. Durch die reproduzierbare Einstellbarkeit der Verbindungen zwischen den einzelnen Formkörpern im erfindungsgemäßen Verfahren ist somit auch die interkonnektierende Porosität, d.h. die Anordnung der Poren und deren Verbindungskanäle, im fertigen porösen Werkstoff sehr fein und reproduzierbar einstellbar, da die Formkörper und deren Verbindungsstege als Platzhalter für die Poren und deren Verbindungen bzw. Durchtrittsöffnungen dienen. Es ist auch sichergestellt, daß im fertigen Werkstoff die Porosität interkonnektierend ist, d.h., daß die einzelnen Poren jeweils durchgehend miteinander in Verbindung stehen. Das erfindungsgemäße Verfahren zeichnet sich gerade durch die Reproduzierbarkeit und Standardisierbarkeit der Herstellung aus, mit dem Ergebnis einer absolut gleichmäßigen und mit einer geringen Streuung behafteten Struktur, sowie durch die weitaus einfachere und kostengünstigere Herstellung des erfindungsgemäßen Werkstoffes. Es hat sich gezeigt, daß der erfindungsgemäße Werkstoff trotz seiner besseren Reproduzierbarkeit und Standardisierbarkeit in einem Bruchteil der Zeit hergestellt werden kann, wie ein Werkstoff gemäß der DE-A-40 33 291.

Die einfache Durchführbarkeit der erfindungsgemäßen Herstellung des Werkstoffes beruht auf der Verwendung von leicht herauslösbaren deformierbaren Formkörpern, die nicht miteinander verbunden werden müssen, sondern einfach gegeneinander gepreßt werden, beispielsweise durch den Einspritzdruck des eingespritzten Materials, wobei sich in Abhängigkeit vom Druck des eingespritzten Materials die Breite bzw. der Querschnitt des "Stegs" zwischen den Formkörpern in dem Formkörperverbund reproduzierbar bestimmen und einstellen läßt, wodurch im fertigen porösen Werkstoff eine standardisierte interkonnektierende Porosität erzeugt werden kann.

Als Material für die deformierbaren Formkörper wird vorzugsweise ein schäumbarer Kunststoff verwendet. Besonders bevorzugt ist ein Schaumstoff aus expandierbarem Polystyrol (EPS), es können aber auch alle anderen geschäumten oder hohlkugelförmigen Kunststoff-Formkörper verwendet werden, die die geeignete Deformierbarkeit und leichte Löslichkeit aufweisen. Die Löslichkeit der Formkörper kann auch durch ihre Porosität (Mikroporosität) gesteuert werden.

Vorzugsweise weisen die deformierbaren Formkörper eine Größe zwischen etwa 200 µm und mehreren mm auf, beispielsweise bis zu 5 mm, besonders bevorzugt eine Größe zwischen 200 µm und 3000 µm, zwischen 500 µm und 3000 µm oder zwischen 1000 µm und 2000 µm. Bevorzugt wird eine bestimmte Größenfraktion von Formkörpern verwendet, die beispielsweise durch Sieben oder aufgrund ihres Herstellungsverfahrens ausgewählt wird, beispielsweise eine Größe von etwa 1000 µm. Durch Wahl der Größe der Formkörper läßt sich die Porosität des fertigen porösen Werkstoffes reproduzierbar, exakt und standardisierbar einstellen, auch kleine Poren bis zu einer Porengröße von weit unter 500 µm, beispielsweise bis zu 200 µm.

Es ist auch möglich, als Ausgangsmaterial des erfindungsgemäßen Verfahrens eine Mischung von Formkörpern verschiedener Größe und/oder verschiedener Morphologie zu verwenden.

Als Material für die Formkörper lassen sich gegebenenfalls auch andere Substanzen verwenden, wie Wachs, Gele, Paraffine, Kollagene, chitinähnliche Substanzen oder Gemische aus solchen Substanzen, gegebenenfalls mit Bindemitteln mit gelartigem Charakter. Die Formkörper können auch aus einem Verbund aus feindispersem Material mit einem leicht löslichen hydrophilen oder hydrophoben Bindemittel bestehen, wobei sich das Bindemittel chemisch leicht herauslösen läßt.

Vorzugsweise weisen die einzelnen Formkörper selbst eine Mikroporosität auf, die zwischen 1 und 40 %, vorzugsweise zwischen 15 und 25 % liegt.

Das Material, das um die deformierbaren Formkörper herum geformt wird, sollte gieß-, spritz- oder schüttbar sein, insbesondere unter Druck. Wenn in der vorliegenden Beschreibung von "gießfähigem" Material die Rede ist, ist darunter jedes gieß-, spritz- oder schüttbare Material zu verstehen. Als gießfähiges Material können die verschiedensten Materialien verwendet werden, je nach Verwendungszweck und je nachdem, ob das gießfähige Material nur eine Zwischenstufe im Herstellungsverfahren bildet und später wieder herausgelöst wird, oder ob das gießfähige Material einen Teil des fertigen Werkstoffes bildet, beispielsweise das Gerüst aus schalenartigen Strukturen (Positiv) oder die miteinander über Stege verbundenen Formkörper des fertigen Werkstoffs (Negativ). Beispielsweise können als Material sowohl für das fertige Positiv als auch für das fertige Negativ Keramik- oder Keramikverbundwerkstoffe verwendet werden, vorzugsweise derartige Materialien, die hohe Dichte aufweisen und einer Sinterung unterzogen werden können. Beispielsweise können derartige Keramikwerkstoffe verwendet werden, die nach der Sinterung sehr leicht vom Körper resorbiert werden. Es können aber auch Keramikmaterialien hoher Dichte verwendet werden, die nach der Sinterung kaum oder nur sehr langsam resorbiert werden. Bevorzugte Keramikmaterialien sind Hydroxylapatit, Tricalciumphosphat und deren Gemische. Hydroxylapatit und Tricalciumphosphat sowie deren Gemische können vorzugsweise mit Bindemitteln, wie Agarose, Agar-Agar, Chitosan oder Gelen versetzt werden, beispielsweise im Verhältnis 10:90 bis 70:30, vorzugsweise etwa 50:50.

Als gießfähiges Material bzw. als Material für das schalenartige Gerüst (Positiv) oder die über Stege verbundenen Formkörper (Negativ) können aber auch verschiedene andere Materialien verwendet werden, beispielsweise gießbare und spritzbare Kunststoffe, die sowohl resorbierbar als auch nicht resorbierbar sein können, Polylaktate, Polyglycolate, resorbierbare Polyaminosäuren sowie deren Gemische, Kollagene oder ähnliche im Körper resorbierbare bzw. auflösbare oder nicht-resorbierbare bzw. -auflösbare Materialien oder Kunststoffe auf PMMA-Basis. Bevorzugt sind auch Metalle, Metall-Legierungen oder Metall-Verbundwerkstoffe sowohl als Positivform mit einem Metalltrabekelgerüst als auch als Negativform mit einem vorzugsweise kugelförmigen Metallformkörperverbund mit Verbindungsstegen. Insbesondere für die Kraftübertragung als Wirbelkörperersatz oder -teilersatz zur Fusion von Wirbelkörpern hat der erfindungsgemäße Werkstoff aus miteinander über Stege verbundenen Formkörpern, vorzugsweise aus Metall, sehr günstige biomechanische Eigenschaften, die beispielsweise günstiger sind als die der in der DE-C-31 06 917 beschriebenen offenporigen oder offenzelligen Metallstruktur. Bei Verwendung von Metall als Material für das dreidimensionale Gerüst wird das Metall vorzugsweise um Formkörper aus Keramik oder Gips ausgebildet. Als Material insbesondere für das dreidimensionale Gerüst des porösen Werkstoffs können auch Silikon, Kautschukderivate oder verwandte Polymere auf Gummibasis verwendet werden.

Grundsätzlich können alle beliebigen Werkstoffe in die Negativ- oder Positivform gebracht werden, beispielsweise können schalenförmige Wabenkonstruktionen reproduzierbar und standardisierbar hergestellt werden, oder es können durch Vermischung verschieden geformter deformierbarer Formkörper als Ausgangsmaterial polymorphe Werkstoffe erzeugt werden, beispielsweise als Implantatwerkstoffe. Beispielsweise können wasserlösliche Formkörper mit schmelzbaren Materialien kombiniert werden oder es können sinterfähige, gießbare Materialverbunde mit wasserlöslichen Formkörpern kombiniert werden oder es können im Spritzgußverfahren verarbeitbare Kunststoffe, Keramikmaterialien oder Metalle, Metall-Legierungen oder Metall-Verbundwerkstoffe in der Weise kombiniert werden, daß jeweils die Formkörper entweder physikalisch oder chemisch oder auf andere Weise wieder auslösbar sind und das dreidimensionale Schalengerüst als Stützgerüst verbleibt. Das Stützgerüst kann anschließend durch physikalische oder chemische Verfahren verfestigt, oberflächenbehandelt oder mechanisch nachbearbeitet werden. Die genannten Metalle, Metall-Legierungen oder Metall-Verbundwerkstoffe können beispielsweise gießbar oder im Schleuderguß oder im Vakuumfeinguß verarbeitbar sein. Wenn beispielsweise ein Keramik-Negativmodell aus Formkörpern mit einem Metallguß in Form eines Schalengerüstes kombiniert wird, so kann dieser Verbundwerkstoff im sogenannten HIP-Verfahren sehr hoch verdichtet werden und ist mechanisch sehr stabil und tragfähig. Auch nach Entfernen der Keramik ist die verbleibende Trabekelstruktur bzw. Schalenstruktur sehr stabil und mechanisch außerordentlich tragfähig, stellt ein sehr leichtes und schnell durchwachsbares Implantat für den Knochen dar und weist einen sehr geringen Materialaufwand auf. Die Formkörper können nach dem Verfestigen des sie umgebenden Materials physikalisch oder chemisch herausgelöst werden, beispielsweise im Wasserbad, in einer Waschmaschine oder durch Lösungsmittel, wobei bei Verwendung von geschäumtem Polystyrol oder einem ähnlichen geschäumten Kunststoff Aceton als Lösungsmittel besonders geeignet ist.

Für diejenigen Materialien, die im Verlauf des Herstellungsverfahrens wieder herausgelöst werden und nicht Bestandteil des fertigen Werkstoffes sind, kommen auch noch andere Substanzen in Betracht, beispielsweise Gips, der besonders einfach und rasch verarbeitbar und außerdem billig ist.

Eine besonders gute Durchdringung der Formkörperschüttung beim Befüllen durch das gießförmige Material ist dann gegeben, wenn die Formkörper hydrophob sind und das gießfähige Material hydrophil ist.

Die Gesamtporosität des fertigen porösen Werkstoffs beträgt vorzugsweise zwischen 50 und 90 %, besonders bevorzugt zwischen 65 und 85 %. Das Material sowohl für das fertige Schalengerüst (Positiv) als auch für die Formkörper des Negativwerkstoffs kann eine Mikroporosität aufweisen, beispielsweise zwischen 1 und 40 %, besonders bevorzugt zwischen 15 und 25

%.

Für bestimmte Anwendungszwecke kann es vorteilhaft sein, die Durchtrittsöffnungen zwischen den einzelnen Poren des interkonnektierenden Porensystems des porösen Werkstoffs variabel zu machen oder zu erweitern. Dies ist insbesondere dann vorteilhaft, wenn Knochenzement in den Werkstoff eingesaugt oder durch diesen durchgesaugt oder wenn ein schnellerer Knocheneinwuchs erreicht werden soll, wobei gegebenenfalls eine niedrigere Druckfestigkeit des Werkstoffs in Kauf genommen werden kann. Um den Querschnitt der Verbindungen bzw. Durchtrittsöffnungen zwischen den einzelnen Poren zu erweitern, wird der Werkstoff mit dem interkonnektierenden Porensystem anschließend chemisch behandelt. Eine besonders vorteilhafte chemische Behandlung ist die Einwirkung einer Säure oder Lauge, die das Porensystem durchströmt. Durch diese chemische Behandlung werden zunächst die dünnen Lamellen des Gerüsts an der Verbindungsstelle zwischen zwei Poren angegriffen und aufgelöst, und damit der Querschnitt der Porenverbindung erweitert. Durch die Wahl des verwendeten chemischen Mittels, die Einstellung von dessen Verdünnung und insbesondere durch die Variation der Einwirkzeit der chemischen Behandlung auf den Werkstoff können die ursprünglichen Porenverbindungen kontrolliert und definiert erweitert und den Erfordernissen angepaßt werden. In vorteilhafter Weise wird so vorgegangen, daß eine verdünnte Säure kurzzeitig den Werkstoff durchströmt, so daß nur die Porenverbindungen aufgeweitet, die Struktur des Gerüsts als solche aber nicht wesentlich angegriffen oder verändert wird. Wenn erfindungsgemäß das ein interkonnektierendes Porensystem aufweisende Gerüst als Ausgangs- oder Zwischenmaterial für einen Werkstoff aus miteinander verbundenen Formkörpern ("Negativ") dient, wird durch eine Vergrößerung der Durchtrittsöffnungen zwischen den Poren des interkonnektierenden Porensystems des Gerüsts eine Vergrößerung des Querschnitts der Verbindungen bzw. "Stege" zwischen den einzelnen Formkörpern bewirkt, nachdem das Porensystem zur Ausbildung der Formkörper mit einem gieß-, spritzoder schüttfähigen Material ausgegossen wird. Damit wird die Festigkeit des fertigen Negativ-Werkstoffs aus miteinander über Stege verbundenen Formkörpern erzielt.

Wenn eine besonders hohe mechanische Stabilität des "Positiv"- oder "Negativ"-Werkstoffs angestrebt wird, können vorteilhafterweise Abschnitte des Werkstoffs in seinem Inneren massiv ausgeführt werden. Dabei kann das interkonnentierende Porensystem des Gerüsts beim Positiv-Werkstoff bzw. die Konfiguration aus verbundenen Formkörpern beim Negativ-Werkstoff im übrigen aufrechterhalten werden. Vorzugsweise sind die massiven Abschnitte im Innern des Werkstoffs in Form massiver Streben, wie Verstärkungsstreben oder Trajektorien ausgebildet. Die massiven Abschnitte sind vorzugsweise etwa zylinderförmig und weisen in ihrem Querschnitt einen Durchmesser von vorzugsweise etwa 0,5 bis 3 mm auf. Die Achse des Zylinders verläuft vorzugsweise in Richtung der voraussichtlichen Hauptbelastungsrichtung des Werkstoffs. Die massiven Abschnitte erstrecken sich vorzugsweise in ihrer Länge durch den gesamten Werkstoff, können sich aber auch nur über einen Teil des Werkstoffs erstrecken. Es können eine oder mehrere derartiger Verstärkungsstreben vorgesehen sein, beispielsweise 2 bis 6, vorzugsweise etwa 3 oder 4 Streben. Die massiven Streben bewirken eine Erhöhung insbesondere der Druckstabilität des Werkstoffs unter Beibehaltung der hohen Porosität und Knochendurchbaubarkeit des Positiv-Werkstoffs.

Um die massiven Abschnitte im "Positiv"-Werkstoff herzustellen, werden bei der Erstbefüllung der Form mit den Formkörpern in die Form Kanülen, wie Befüllungskanülen, eingebracht, um die herum die Formkörper angeordnet werden. Anschließend wird die Form durch die Kanülen und/oder wie üblich von außerhalb mit dem gießfähigen Material gefüllt. Während der Befüllung werden die Kanülen sukzessive aus der Form herausgezogen, wobei im Werkstoff neben dem ein interkonnektierendes Porensystem aufweisenden Gerüst massive Abschnitte an denjenigen Stellen in der Form entstehen, die zuvor von den Kanülen eingenommen worden sind. Das Herausziehen der Kanülen und die Befüllung mit dem gießfähigen Material werden dabei zeitlich koordiniert. Hierbei entstehen das Gerüst aus den schalenartigen Strukturen und die massiven Abschnitte in einem Guß, so daß der gesamte Werkstoff aus einem homogenen Material besteht.

Bei der Herstellung des "Negativ"-Werkstoffs mit massiven Abschnitten werden beim Befüllen der Form mit den Formkörpern ebenfalls Platzhalter, beispielsweise in Form von Röhrchen, in die Form eingebracht. Diese Röhrchen können entweder hohl oder massiv sein und weisen in jedem Fall dieselbe äußere Form und Gestaltung auf wie die später in dem Werkstoff auszubildenden massiven Abschnitte. Beim anschließenden Befüllen der Form mit dem gießfähigen Material werden die Röhrchen umschlossen. Anschließend werden die als Platzhalter dienenden Röhrchen entfernt, beispielsweise durch Herausziehen aus der Form oder durch chemisches oder physikalisches Herauslösen. Dabei hinterlassen die Platzhalter Hohlräume innerhalb des hergestellten Gerüsts. Diese Hohlräume werden beim anschließenden Ausgießen des Gerüsts mit dem die Formkörper bildenden gießfähigen Material zu massiven Abschnitten aufgefüllt. Auch bei diesem Verfahren entstehen somit die massiven Abschnitte und der umgebende Werkstoff aus miteinander verbundenen Formkörpern in einem Guß, so daß der gesamte Werkstoff aus einem homogenen Material besteht. Es ist aber sowohl beim Positiv- als auch beim Negativ-Werkstoff auch möglich, die massiven Abschnitte aus einem unterschiedlichen Material auszubilden, wenn dies beispielsweise aus Festigkeitsgründen bevorzugt ist. Beispielsweise können in die massiven Abschnitte Fasern, z.B. aus Keramik, Kohle, Glas oder Metall, zur Verstärkung eingebracht werden. Die Matrix des Werkstoffs der massiven Abschnitte kann dabei aus demselben Material oder aus einem anderen Material bestehen wie das umgebende Gerüst bzw. die Formkörper. Um die massiven Abschnitte aus einem anderen Material wie das umgebende Gerüst bzw. die Formkörper auszubilden, werden die massiven Abschnitte in einem getrennten Verfahrensschritt hergestellt. Beispielsweise werden die Platzhalter zunächst noch in der Form belassen, bis der Guß des Werkstoffes ausgeführt ist, und erst anschließend mit einem anderen Material aufgefüllt. Besonders bevorzugt sind beispielsweise massive Abschnitte in Form von Streben aus einem Metall, die in einem Negativ-Werkstoff aus Keramik-Formkörpern eingebettet sind, um die Biegefestigkeit zu erhöhen. Dabei schrumpft die Keramik beim abschließenden Sintern auf das Metall auf, wobei Keramik und Metall fest miteinander verbunden werden.

Die äußere Form des fertigen Werkstoffes ist beliebig wählbar und wird insbesondere durch die Gestaltung der beim erfindungsgemäßen Verfahren verwendeten Gießform bestimmt. Durch die Variation des Ausgangsfüllvolumens der Gießform mit den Formkörpern und der Zahl und Richtung der Einspritzkanäle des gießfähigen Materials können an einer oder mehreren Außenflächen des Werkstoffes auch gezielt massive Abschnitte ausgebildet werden, beispielsweise in Form eines dünnen massiven Deckels. Besonders bevorzugt ist ein sogenannter tricorticaler Quader, d.h. ein Quader aus einem porösen Werkstoff, bei dem an zwei gegenüberliegenden Flächen des Quaders und an der diese beiden Flächen verbindenden Fläche eine massive, dünne Außenschicht ausgebildet ist. Diese massive Schicht kann beispielsweise eine Dicke von 0,5 bis 3 mm, vorzugsweie mindestens 1 mm aufweisen. Derartige tricorticale Quader sind besonders vorteilhaft bei der Verwendung als Knochenersatzwerkstoff oder Kunstknochen, beispielsweise zum Knochenersatz im Becken.

Vorzugsweise enthält der fertige Werkstoff mindestens einen Wirkstoff, insbesondere bei Verwendung des Werkstoffes in der Medizintechnik, beispielsweise für die Herstellung von Implantaten und Knochenersatzwerkstoffen, als Filtersystem oder als Wirkstoffträger in Form eines sogenannten "drug delivery system". Der fertige Werkstoff enthält vorzugsweise 0,01 bis 10 % des Wirkstoffes, der aus dem Werkstoff bevorzugt protrahiert freisetzbar ist. Beispielsweise kann das Gerüst des Positivs bzw. der über Stege verbundene Kugelverbund des Negativs schichtweise ein- oder mehrlagig mit einem oder mehreren Wirkstoffen beschichtet sein. Es kann auch die Makro- oder Mikroporosität des Werkstoffes reproduzierbar mit einem Gemisch aus einem Bindemittel und einem Wirkstoff aufgefüllt sein, wobei der Wirkstoff vorzugsweise über eine definierbare Zeit protrahiert im Körper freisetzbar ist.

Die zugesetzten Wirkstoffe können gezielt den Gefäß- oder Knocheneinwuchs induzieren, gezielt einen lokalen Tumor therapieren oder gezielt zu einer Infektsanierung beitragen. Die erfindungsgemäß erreichbaren Konzentrationen liegen weit über denen eines Wirkstoffes, beispielsweise eines Antibiotikums, die bei systemischer Behandlung erreicht werden könnten. Durch geeignete Kombination mit Bindemitteln oder durch Veränderungen der Porosität kann die Verzögerung der Wirkstoff-Freisetzung eingestellt werden.

Als Wirkstoffe können beispielsweise Gentamycin, Glindamycin, ein Gyrasehemmer oder ein anderes Antibiotikum oder eine Kombination von zwei oder mehreren verschiedenen Antibiotika verwendet werden. Als Wirkstoff kann auch ein das Wachstum induzierender Wirkstoff verwendet werden, beispielsweise vom Typ eines "Bone morphogenetic protein", eines Wachstumsfaktors oder eines anderen chemotaktisch oder hormonell wirkenden Faktors, der das Einsprossen von Gefäßen oder die Differenzierung von knochenbildenden Zellen bewirkt oder auch den Abbau des Knochens verhindert. Als Wirkstoff können ferner ein Cytostatikum, eine Kombination mehrerer Cytostatika oder auch eine Kombination von Cytostatika mit anderen Wirkstoffen, beispielsweise Antibiotika oder Hormonen, verwendet werden.

Das Makroporensystem des porösen Werkstoffes kann auch mit Aktivkohle oder einem anderen hochporösen Adsorbens aufgefüllt werden.

Ferner kann dem Werkstoff ein Füller zugesetzt werden, vorzugsweise in Form von Füllerpartikeln. Als Füllerpartikel können 1 bis 95 %, vorzugsweise 1 bis 80 % Tricalciumphosphat oder Hydroxylapatit oder ein Gemisch aus beiden oder eine andere Calciumphosphatverbindung oder Calciumverbindung verwendet werden. Die Partikelgröße beträgt vorzugsweise 20 bis 300 μm, besonders bevorzugt 50 bis 250 μm, und das Porenvolumen der porösen Füllerpartikel beträgt vorzugsweise 0,1 bis 0,8 ml/g. Der Zusatz von Füllerpartikeln ist besonders bei Verwendung spritzbarer Kunststoffe bevorzugt. Wenn der Werkstoff aus Kunststoff oder Metallen besteht, ist auch eine Beschichtung mit Tricalciumphosphat und/oder Hydroxylapatit wünschenswert. Dies ist insbesondere dann von Vorteil, wenn ein knöchernes Durchwachsen des Werkstoffes gewünscht wird. Unter Belastung setzt der Knochen die Deformationsenergie im Interface an glatten Implantaten in Relativbewegung um, was zur Knochenresorption führt, während bei rauhen Oberflächen, beispielsweise derart beschichteten Oberflächen, keine derartige Relativbewegung auftritt.

Als Füller kann auch Aktivkohle oder ein anderes stark gasund/oder flüssigkeitabsorbierendes Material verwendet werden, vorzugsweise in einer Konzentration zwischen 1 und 80 % bzw. 5 und 80 %.

Folgende Verfahren sind erfindungsgemäß besonders bevorzugt:

Ein Verfahren zur Herstellung eines Werkstoffs mit einem dreidimensionalen Schalengerüst mit den folgenden Verfahrensschritten: Aneinanderpressen von vorzugsweise kugelförmigen deformierbaren Formkörpern zu einer dreidimensionalen Formkörperpackung, Formen eines sich vom Material der Formkörper unterscheidenden Materials um die Formkörper herum unter Verformung der Formkörper zur Ausbildung eines dreidimensionalen Schalengerüsts und Entfernen der Formkörper, so daß lediglich das dreidimensionale Schalengerüst verbleibt und den Werkstoff bildet.

Ein Verfahren zur Herstellung eines Werkstoffs mit miteinander über Stege verbundenen Formkörpern mit den folgenden Verfahrensschritten: Bildung einer dreidimensionalen Formkörperpackung durch Aufeinanderpressen von deformierbaren Formkörper, Formen eines dreidimensionalen Gerüsts aus einem Wachs oder einem leicht schmelzbaren Polymer um die Formkörper herum, Aushärten des Gerüsts aus Wachs oder dem leicht schmelzbaren Polymer, chemisches Herauslösen der Formkörper, Füllen des durch Entfernen der Formkörper entstandenen durchgehenden Hohlraumsystems mit einer Keramikmasse und Entfernen des Gerüst aus Wachs oder dem leicht schmelzbaren Polymer durch Anwendung von Hitze. Bei diesem Verfahren werden in den Hohlräumen vorzugsweise Keramikkugeln gebildet. Die Keramikkugeln können durch Anwendung von Hitze in β- und α-Whitlockit-Tricalciumphosphatverbindungen umgewandelt werden oder können im Sinterverfahren zu festen Kugelkonglomeratkörpern zusammengeschweißt werden, beispielsweise aus Hydroxylapatit.

Ein Verfahren zum Herstellen eines Metallwerkstoffs aus einem Schalengerüst, mit den folgenden Verfahrensschritten:

Herstellung einer Schüttung aus deformierbaren Kunststoffkugeln, z.B. aus Polystyrol, Aufeinanderpressen der Kugeln und Ausfüllen mit Wachs, Auslösen der Kunststoffkugeln mit einem Lösungsmittel, beispielsweise Aceton, Trocknung des Wachsgerüstes, Ausgießen des Wachsgerüstes mit einer keramischen Masse, beispielsweise Tricalciumphosphat und Bindemittel in einem gelartigen Gemisch, Auslösen des Wachses im Ölbad mit Verfestigung der Keramikmasse, anschließende Sinterung der Keramikmasse im Ofen mit einer Erwärmungsgeschwindigkeit von etwa 1°C/min bis zu einer Temperatur von etwa 1300°C über ca. 24 Stunden, Einbringen des entstehenden Keramikkörpers in einen Muffelofen und Ausgießen in einem Schleudergußverfahren mit einer CoCrMo-Legierung, Sandstrahlen des Keramik-Metall-Verbundes und Herauslösen der Keramik mit Säure, beispielsweise Salzsäure.

Ein Verfahren zum Herstellen eines Metallwerkstoffs aus miteinander über Stege verbundenen Formkörpern mit den folgenden Verfahrensschritten: Ausbildung einer Schüttung aus deformierbaren Kunststoffkugeln, beispielsweise geschäumten Polystyrolkugeln, Kompression der Kugeln, Ausfüllen des Volumens um die Kugeln mit einer Keramikmasse, Auslösen der Kugeln mit einem Lösungsmittel, beispielsweise Aceton, Trocknung, vorzugsweise in einer aufsteigenden Acetonreihe, Sintern des entstandenen keramischen Schalengerüsts im Ofen mit einer Aufheizgeschwindigkeit von etwa 1°C/min bis zu einer Temperatur von 1300°C über ca. 24 Stunden, Einbringen des Keramikgerüsts in einen Muffelofen und Auffüllen des Makroporenvolumens mit einer Metallegierung, vorzugsweise CoCrMo, im Schleuderguß- oder Feinguß-Vakuumverfahren, Auslösen der Keramik im Säurebad, beispielsweise in Salzsäure.

Beim erfindungsgemäßen Verfahren stellt sich nach dem Befüllen der Form mit dem gieß-, spritz- oder schüttfähigen Material vorzugsweise ein isostatischer Druck in der Form ein. Beim Befüllen der Form mit dem gieß-, spritz- oder schüttfähigen Material wird vorzugsweise ein Endabschnitt an mindestens einem Ende der Form vollständig mit dem Material befüllt, so daß dieser Endabschnitt einen nicht-porösen Abschnitt des fertigen Werkstoffs bildet. Die deformierbaren Formkörper bestehen vorzugsweise aus einem schäumbaren Kunststoff, wie Polystyrol, wobei das Raumgewicht des schäumbaren Kunststoffes, z.B. des Polystyrols, vorzugsweise etwa 17 bis 70 g/l beträgt. Die deformierbaren Formkörper sind im nicht-deformierten Zustand vorzugsweise kugelförmig, elliptisch, granular oder vieleckig; vorzugsweise weisen die Formkörper eine Größe zwischen etwa 200 μm und 3000 μm auf. Als Ausgangsmaterial wird vorzugsweise eine Mischung von Formkörpern verschiedener Größe und/oder verschiedener Morphologie verwendet. Das Material für das Gerüst bzw. für die Formkörper des fertigen Werkstoffs besteht vorzugsweise aus einer Keramikmasse, besonders bevorzugt aus Hydroxylapatit und/oder Calciumphosphat. Das Material für das Gerüst bzw. für die Formkörper des fertigen Werkstoffs besteht vorzugsweise aus Metall oder einer Metallegierung. Die Gesamtporosität des porösen Werkstoffs beträgt vorzugsweise zwischen 50 und 90 %, besonders bevorzugt zwischen 65 und 85 %. Das Material für das Gerüst (Positiv) bzw. für die Formkörper (Negativ) weist vorzugsweise eine Mikroporosität auf, wobei die Mikroporosität zwischen 1 und 40 %, vorzugsweise zwischen 15 und 25 % beträgt. Das gieß-, spritz- oder schüttfähige Material enthält vorzugsweise ein Bindemittel, und das Material kann durch Sinterung verfestigt werden. Die Formkörper sind vorzugsweise hydrophob, und das gieß-, spritz- oder schüttfähige Material ist vorzugsweise hydrophil. Das Material der Formkörper und/oder des Gerüsts kann einen Wirkstoff enthalten, der vorzugsweise protrahiert freisetzbar ist. Die Makro- und/oder Mikroporosität des Werkstoffes kann zumindest teilweise mit einem Gemisch aus einem Wirkstoff und einem Bindemittel aufgefüllt werden, wobei der Wirkstoff vorzugsweise protrahiert freisetzbar ist. Dem Material des Werkstoffs kann ferner ein Füller zugesetzt werden.

Die Form kann in ihrem Innern Platzhalter aufweisen, die während des Befüllens der Form mit dem gieß-, spritz- oder schüttfähigen Material sukzessive aus der Form herausgezogen werden, wobei der beim Herausziehen der Platzhalter entstehende Raum mit nicht-porösem Material gefüllt wird, so daß im Werkstoff massive Abschnitte entstehen; hierbei können die Formkörper gegebenenfalls im wesentlichen nicht deformierbar sein. Die Platzhalter sind vorzugsweise Kanülen, und die massiven Abschnitte sind in dem Werkstoff vorzugsweise in Form von Streben, wie Verstärkungsstreben oder Trajektorien enthalten. Die Form kann auch Platzhalter in ihrem Inneren aufweisen, die nach dem Befüllen der Form gemäß Verfahrensschritt (b) der Ansprüche entfernt werden, so daß an der Stelle der Platzhalter Hohlräume in dem Werkstoff entstehen; auch hierbei können die Formkörper gegebenenfalls im wesentlichen nicht deformierbar sein. Die Hohlräume können gegebenenfalls beim Ausgießen des Gerüsts gemäß Verfahrensschritt (d) der Ansprüche ebenfalls ausgefüllt werden und nach dem Entfernen des Gerüstmaterials gemäß Verfahrensschritt (e) als massive Abschnitte im Werkstoff verbleiben. Diese massiven Abschnitte des Werkstoffs können die Form von Streben, wie Verstärkungsstreben oder Trajektorien aufweisen.

Die Verbindungen zwischen den Poren des interkonnektierenden Porensystems können anschließend durch chemische Behandlung vergrößert werden, z.B. durch die Einwirkung von Säure und/oder Lauge, wobei die Vergrößerung der Porenverbindungen durch die Wahl der verwendeten Säure und/oder Lauge, deren Verdünnung und die gewählte Einwirkungszeit gesteuert werden kann.

Der fertige Werkstoff kann in seiner Struktur massive Abschnitte, wie Verstärkungsstreben oder Trajektorien aufweisen, wobei die Streben vorzugsweise im wesentlichen zylinderförmig sind und vorzugsweise Durchmesser zwischen 0.5 und 3 mm aufweisen. Der Werkstoff kann durch chemische Behandlung erweiterte Verbindungen oder Öffnungen zwischen den einzelnen Poren des Porensystems aufweisen. Vorzugsweise weist der fertige Werkstoff die Form eines tricorticalen Quaders auf.

Die Erfindung wird nachstehend anhand von Ausführungsbeispielen und der Zeichnungen näher erläutert. Es zeigen:

Fig. 1 - 3    das Einfüllen von deformierbaren Formkörpern in eine Form und das Befüllen der Form mit einem gießfähigen Material,

Fig. 4 - 6    elektronenmikroskopische Aufnahmen des fertigen Werkstoffs,

Fig. 7 - 10    Seitenansichten und Aufsichten des fertigen Werkstoffs,

Fig. 11    eine Ansicht eines fertigen Werkstoffs in Form eines tricorticalen Quaders, und

Fig. 12    eine schematische Ansicht einer Gießform zur Herstellung eines tricorticalen Quaders.

Ein besonders bevorzugtes Ausgangsmaterial für die deformierbaren Formkörper ist Styropor®, beispielsweise Styropor® F414, d.h. ein Schaumstoff aus expandierbarem Polystyrol (EPS), das mit Pentan als Treibmittel aufgeschäumt ist. Bevorzugte Raumgewichte des aufgeschäumten Polystyrols sind zwischen 17 g/l und 70 g/l, vorzugsweise etwa 20 g/l bis 35 g/l. Die Korngrößenverteilung des aufgeschäumten Materials liegt zwischen 200 μm und 3000 μm.

Um ein Maß für eine geeignete Deformierkeit der deformierbaren Formkörper zu gewinnen, wurden Versuche zur Bestimmung eines E-Modulähnlichen Parameters für verschiedene Formkörper durchgeführt. Hierfür wurden die entsprechenden Formkörper in einem zylindrischen Gefäß aufgeschüttet bis auf eine Ausgangshöhe von 84,3 mm. Danach wird über einen Stempel eine Kraft F auf die Schüttung ausgeübt und die Kompression, d.h. die Änderung der Ausgangshöhe, in Abhängigkeit von der aufgebrachten Kraft registriert. Über die Formel

$$E = \frac{F \times l}{A \times l}$$

wird dann eine E-Modul-ähnliche Größe bestimmt, wobei

E :    E-Modul-ähnliche Größe
F :    Kraft in N
A :    Fläche des Zylinders in mm$^2$
l :    Ausgangshöhe (84,3 mm)
l :    Änderung der Ausgangshöhe in mm.

Die Versuche ergaben für verschiedene geschäumte Polystyrole, die sich beispielsweise im Raumgewicht, in der Art des Aufschäumens und in der Partikelgröße unterscheiden, Werte für E von etwa 0,5 bis etwa 1,2 N/mm$^2$. Eine derartige Elastizität ist besonders bevorzugt. Die Elastizität kann jedoch auch etwa um den Faktor 10 kleiner oder größer sein. Im Gegensatz dazu beträgt beispielsweise die Größe E für verschiedene Zuckerarten etwa 20 bis 180 N/mm$^2$, wobei jedoch die Zuckerkörper beim Aufbringen der Kraft zum Teil zerdrückt werden.

Als gießfähiges Material wird vorzugsweise eine Mischung aus Hydroxylapatit-Pulver mit einer Agar-Agar-Lösung im Verhältnis 10 g Pulver/7 ml bis 25 ml Agar-Agar-Lösung, vorzugsweise 10 g Pulver/20 ml Agar-Agar-Lösung verwendet. Die Agar-Agar-Lösung besteht vorzugsweise aus einer Mischung von Aqua bidest. mit Agar-Agar im Verhältnis 20 ml bis 50 ml/1 g, vorzugsweise im Verhältnis 40 ml/1 g.

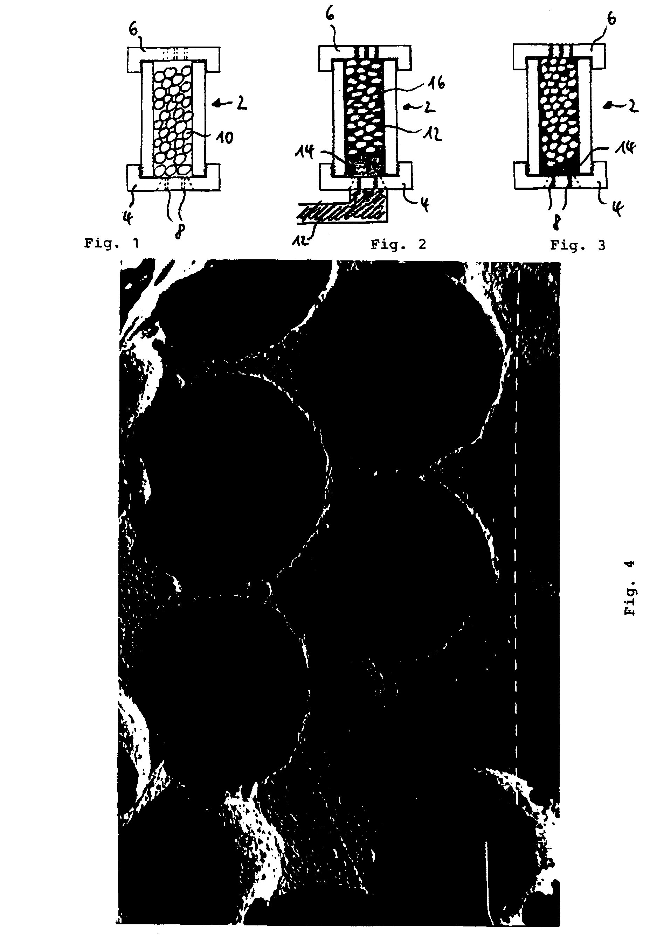

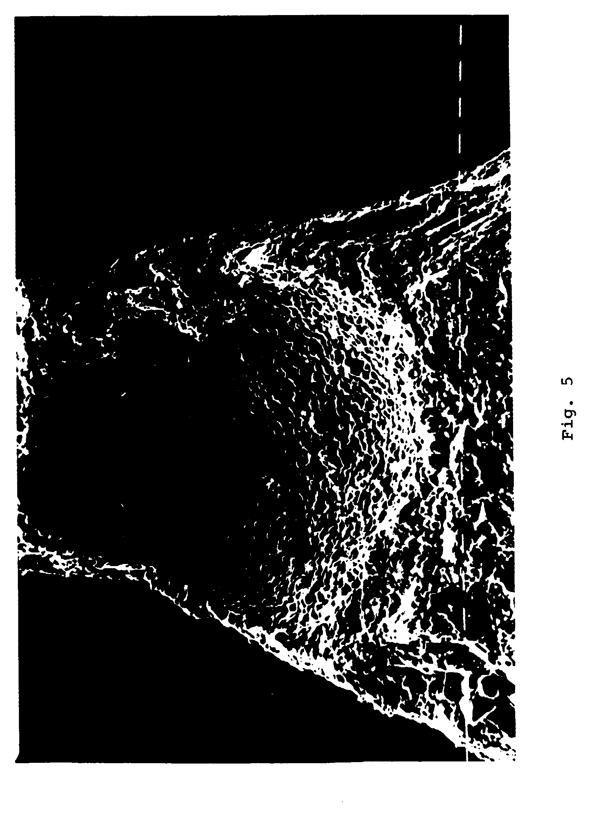

Fig. 1 zeigt schematisch eine Gießform 2 mit Boden 4 und Deckel 6 sowie Füllkanälen 8. In die Form 2 werden geschäumte Polystyrol-Formkörper 10 in Kugelform eingefüllt. Beim Einfüllen der Formkörper in die Gießform 2 wird diese nur soweit gefüllt, daß die Formkörper keinem Druck ausgesetzt sind und nicht verformt werden. Danach wird die Form 2 von unten her mit einer keramischen Masse 12 als gießfähiges Material mit der vorstehenden Zusammensetzung gefüllt, wie in Fig. 2 dargestellt. Aufgrund der Viskosität der keramischen Masse wird zunächst auf die EPS-Formkörper ein in axialer Richtung nach oben wirkender Druck ausgeübt, durch den die Formkörper aneinandergepreßt werden. Die dabei auftretende Volumenverringerung führt dazu, daß am Boden 4 der Form 2 eine polystyrolfreie, nur mit keramischer Masse gefüllte Zone 14 entsteht, die später einen dichten bzw. massiven Deckel des fertigen porösen Werkstoffes bildet. Je nach Ausgangsfüllvolumen der Gießform 2 und Viskosität der keramischen Masse kann die Höhe der Zone 14 und damit die Stärke des Deckels des fertigen Werkstoffes variiert werden. Das Ausgangsfüllvolumen kann aber auch so gewählt werden, daß der gesamte gebildete Werkstoff porös ist und kein massiver Deckel entsteht.

Durch den beim Einfüllen der keramischen Masse unidirektional ausgeübten Druck werden die deformierbaren EPS-Kugeln 10 wahrscheinlich zunächst zu ovalen Körpern geformt, wie z.B. anhand des Körpers 16 in Fig. 2 dargestellt. Nach Abschluß des Einfüllvorgangs bildet sich jedoch in der Form ein in alle Richtungen gleich großer, d.h. isostatischer Druck aus. Dieser führt zusammen mit der Elastizität der EPS-Formkörper und der noch weichen keramischen Masse zu einer Relaxation der verformten EPS-Formkörper in ihre Kugelform. Die Situation bei Abschluß des Einfüllvorgangs ist in Fig. 3 dargestellt. Nach der Verfestigung der keramischen Masse kann eine isotrope Verteilung kugelförmiger EPS-Formkörper beobachtet werden. Nach dem Herauslösen der EPS-Formkörper, beispielsweise durch Aceton, weist der verbleibende Keramikwerkstoff eine durchgehend homogene Struktur mit interkonnektierenden kugelförmigen Poren auf. Die Form des Porensystems und dessen Verbindungskanälen entspricht der Form des Formkörperverbundes mit Verbindungsstegen. Die Durchgangsöffnungen zwischen den Poren können anschließend noch durch chemische Behandlung, beispielsweise mit einer Säure oder Lauge, erweitert werden.

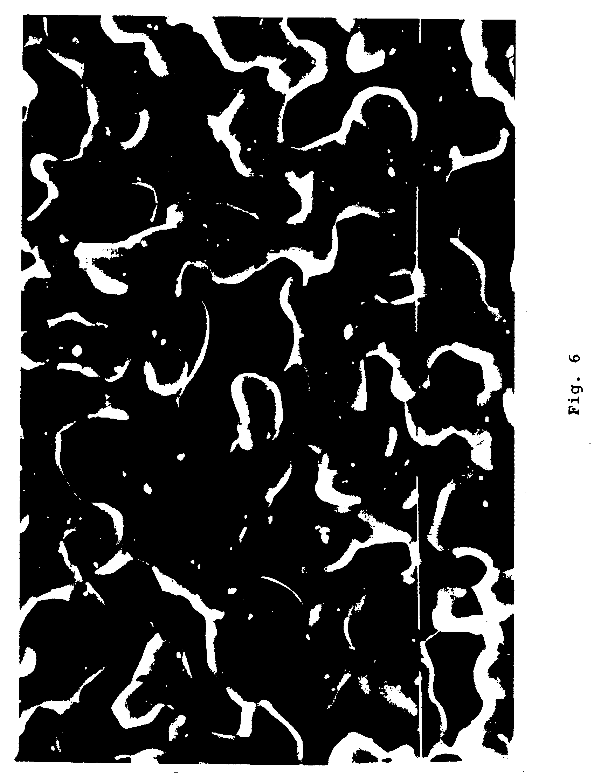

Fig. 4 zeigt eine elektronenmikroskopische Aufnahme eines fertigen Werkstoffs mit einem dreidimensionalen Gerüst, wobei die Schalenform des Gerüsts gut erkennbar ist. Ein Strich in Fig. 4 entspricht 100 μm.

Fig. 5 zeigt einen Ausschnitt einer derartigen Schale in elektronenmikroskopischer Aufnahme, wobei in Fig. 5 ein Strich 10 μm darstellt.

Fig. 6 zeigt schließlich in weiterer Vergrößerung die Oberflächenstruktur. Ein Strich in Fig. 6 bedeutet ebenfalls 10 μm.

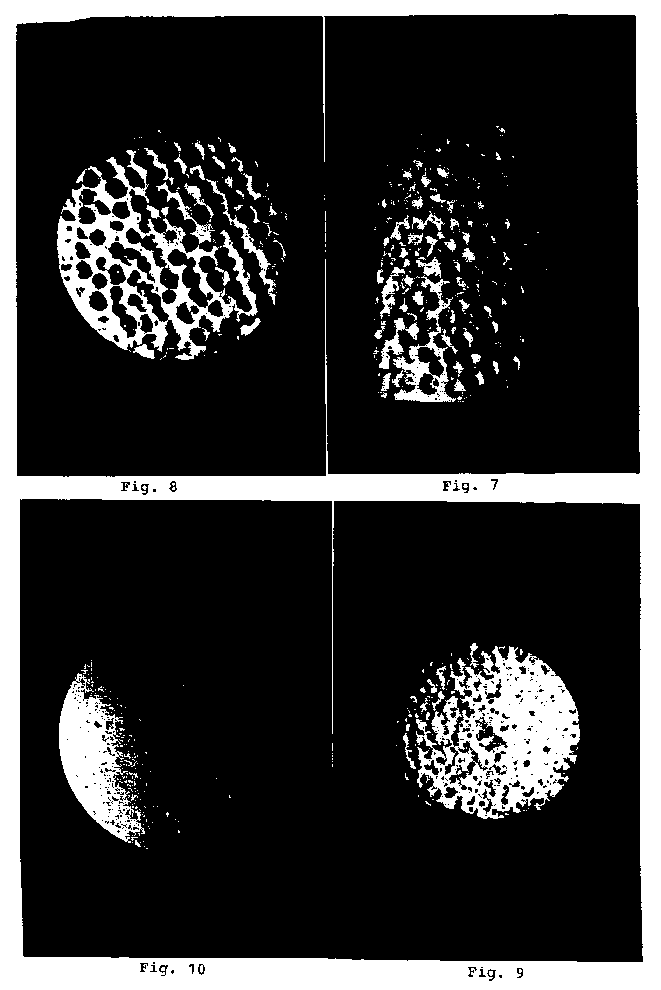

Fig. 7 zeigt in etwa 5- bis 6-facher Vergrößerung eine Seitenansicht eines Zylinders aus einem relativ grobporigen, porösen Positiv-Werkstoff mit einem schalenartigen Gerüst und einem interkonnektierenden, isotropen Porensystem.

Fig. 8 zeigt eine Aufsicht auf einen derartigen Zylinder. Aus den Figuren 7 und 8 ist die homogene, isotrope Verteilung des interkonnektierenden Porensystems sowie das Trabekel- oder Schalengerüst gut erkennbar, das der Knochenstruktur nachempfunden ist.

In Fig. 9 ist eine Aufsicht auf einen Zylinder eines relativ feinporigen "Positiv"-Werkstoffes dargestellt. Auch hier ist das schalenartige Gerüst und das dazwischenliegende interkonnektierende, isotrope Porensystem gut erkennbar.

Fig. 10 zeigt eine Aufsicht auf einen dichten bzw. massiven Deckel eines fertigen "Positiv"-Werkstoffs. Wie vorstehend anhand von Fig. 2 erläutert, läßt sich ein derartiger dichter bzw. massiver Deckel (Abdeckelung) dadurch erzielen, daß beim Befüllen der Form mit dem gießfähigen Material ein Endabschnitt der Form vollständig mit dem gießfähigen Material befüllt wird und die als Platzhalter für das Porensystem dienenden deformierbaren Formkörper vollständig aus diesem Endabschnitt verdrängt werden. Ein Werkstoff mit einem derartigen Deckel ist z.B. besonders vorteilhaft als Plug in einem Markkanal anwendbar. Es ist selbstverständlich durch geeignete Maßnahmen bei der Herstellung auch möglich, beide Endabschnitte oder verschiedene Abschnitte des Werkstoffvolumens, beispielsweise auch entlang der Mantelfläche bei einem zylindrisch ausgebildeten Werkstoff, mit einer derartigen Abdeckung zu versehen, so daß der Werkstoff in diesem Bereich nicht porös, sondern dicht bzw. massiv ist.

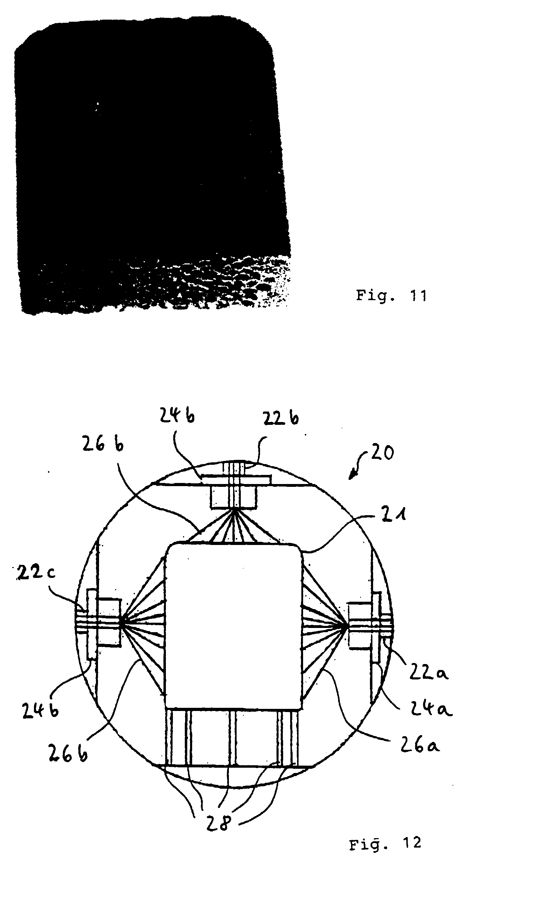

Fig. 11 zeigt in etwa 2- bis 3-facher Vergrößerung einen tricorticalen Quader mit einem porösen "spongiösen" Innenteil, wobei zwei gegenüberliegende Flächen und eine diese verbindende Außenfläche des Quaders eine dünne massive Schicht aufweisen. Derartige tricorticale Quader weisen ausgezeichnete Druckfestigkeit auf. Die Druckfestigkeit wurde an tricorticalen Quadern mit einer Grundfläche von 15 x 15 mm und einer Höhe von 7 mm gemessen. Die Versuche wurden auf einer Universal-Prüfmaschine Instron 1000 bei einer Belastungsgeschwindigkeit von jeweils 5 mm/min durchgeführt. Ein erfindungsgemäßer vollständig poröser Keramikquader mit diesen Abmessungen weist eine Druckfestigkeit von etwa 2 bis 4 MPa auf. Ein tricorticaler Quader mit drei massiven Außenflächen, der ferner zur Porenerweiterung 20 Sekunden lang in 25%iger HCl behandelt wurde, weist eine Druckfestigkeit von 12,2 MPa auf. Ein weiterer tricorticaler Quader mit teilmassiver Innenstruktur, d.h. mit massiven Verstärkungsstreben, weist bei denselben Abmessungen eine Druckfestigkeit von 26,2 MPa auf.

Fig. 12 zeigt schematisch eine Spritzgußform 20 mit einem Innenteil 21 zur Herstellung eines tricorticalen Quaders. Die Einspritzung erfolgt gleichzeitig dreiseitig über Einspritzkanäle 22a, b, c und Verteileranschlüsse 24a, b, c. Mit den Bezugzeichen 26a, b, c ist angedeutet, daß die Einspritzung jeweils fächerartig verteilt auf die drei Beaufschlagungsflächen erfolgt, wo die massiven Außenflächen des tricorticalen Quaders ausgebildet werden. Auf der "freien" Seitenfläche sind Entlüftungsbohrungen 28 vorgesehen.

**Beispiel 1**

**Herstellung eines Werkstoffs (Negativ) aus Keramik zur Verwendung als Knochenersatzwerkstoff-Hohlkörperimplantat mit reproduzierbarer Makro- und Mikroporosität**

In einer zylindrischen Form werden Kugeln aus aufgeschäumtem Polystyrol (EPS), Z.B. Styropor® F 414, mit einer ausgesiebten Größe von etwa 1000 µm aufgeschüttet. Mittels einer aufgesetzten Spritze wird auf 75°C erwärmtes Wachs unter Druck eingespritzt. Nach Aushärten des Wachses wird das Polystyrol mit Aceton herausgelöst, anschließend das Wachsgerüst an der Luft getrocknet und danach mit einer keramischen Masse aufgefüllt. Die Keramikmasse setzt sich z.B. zusammen aus 5 g Tricalciumphosphat oder Hydroxylapatit oder einem Gemisch aus beiden zusammen mit 7 ml Lösung, wobei die Lösung aus 2 g Agar-Agar auf 80 ml Wasser bestehen kann. Der Wachs/Keramik-Rohling wird danach im Ölbad bei Temperaturen um 100°C vom Wachs befreit, und die verbleibende Keramikmasse wird über 6 bis 7 Stunden gefestigt. Der so gewonnene Grundkörper wird im Keramikofen mit einer Aufheizrate von 1°C/Minute bis 1300°C über ca. 24 Stunden gesintert und anschließend über weitere 24 Stunden in Schritten gekühlt. Das so entstandene Keramikimplantat aus Tricalciumphosphat, Hydroxylapatit oder einem Gemisch aus beiden stellt ein sogenanntes "Negativ-Implantat" dar, mit einem über breite Stege verbundenen dreidimensionalen Kugelverbund. Ein derartiges Implantat ist für hohe Kraftübertragung besonders günstig und weist eine für poröse Keramiken außerordentlich hohe Druckstabilität von bis zu 10 MPa auf.

**Beispiel 2**

**Herstellung eines porösen Werkstoffs (Positiv) aus Keramik zur Verwendung als Knochenersatzwerkstoff mit einem durchgehend interkonnektierenden Porensystem und trabekulärer Struktur**

In einen zylindrischen Gefäß werden Kugeln aus aufgeschäumten Polystyrol (EPS), z.B. Styropor® F 414, mit einem Durchmesser von etwa 1000 µm aufgeschüttet und gerüttelt. Mittels einer aufgesetzten Spritze wird unter Druck eine Keramikmasse eingespritzt, die die Zusammensetzung gemäß Beispiel 1 aufweisen kann, und anschließend evakuiert. Der Keramik-Polystyrol-Rohling wird anschließend für eine halbe Stunde im Kühlschrank gehärtet, danach entnommen und im Acetonbad von den Polystyrolkugeln befreit. Nach Trocknung über Aceton an Luft wird der Grünling über ca.24 Stunden mit einer Aufheizrate von 1°C/min im Keramikofen bis zu einer Temperatur von 1300°C gesintert. Die Abkühlung erfolgt schrittweise über weitere 24 Stunden. Danach ist das Keramikimplantat, welches durchgehend gleichmäßige interkonnektierende Poren enthält und trabekulär bzw. schalenförmig strukturiert ist, fertiggestellt.

**Beispiel 3**

**Herstellung eines porösen Werkstoffs (Positiv) aus Metall zur Verwendung als spongiosa-strukturiertes Metallimplantat**

In ein Gefäß werden Kugeln aus geschäumtem Polystyrol (EPS), z.B. Styropor® F 414, aufgeschüttet und gerüttelt. Das zwischen den Kugeln verbleibende Volumen wird mit Wachs aufgefüllt. Mittels einer aufgesetzten Spritze wird das Wachs dabei bei einer Temperatur von 75°C unter Druck eingepreßt. Anschließend wird das Wachsgerüst ausgehärtet, das Polystyrol im Acetonbad ausgelöst und anschließend das verbleibende Wachsgerüst getrocknet. Danach wird das Wachsgerüst (Positiv) mit einer keramischen Masse ausgegossen, beispielsweise mit Tricalciumphosphat, einem Gemisch aus Tricalciumphosphat und Hydroxylapatit oder reinem Hydroxylapatit in einem Gemisch mit einer gelartigen Substanz als Bindemittel; die Keramikmasse kann beispielsweise die Zusammensetzung gemäß Beispiel 1 aufweisen. Im Anschluß daran wird das Wachs im Ölbad bei 100°C ausgelöst und die verbleibende Keramikmasse (Negativ) über 6 bis 7 Stunden verfestigt. Der so hergestellte Grünkörper wird im Keramikofen mit einer Aufheizrate von 1°C/Minuten über ca. 24 Stunden bis 1300°C gesintert und anschließend vorsichtig über weitere 24 Stunden abgekühlt. Der so entstandene Keramikkörper aus Kugeln mit Verbindungsstegen mit definiertem Querschnitt wird in einer Form in einen Muffelofen eingebracht, und im Schleuderguß- oder Feinguß-Vakuumverfahren bei 1000°C mit einer CoCrMo-Legierung ausgegossen. Nach Herausnahme des entstehenden Keramik-Metall-Verbundes wird dieser sandgestrahlt und anschließend im Salzsäurebad von der Keramik befreit. Das Implantat zeigt eine durchgehende trabekuläre bzw. schalenförmige Struktur mit einem dazwischen ausgebildeten standardisierten und reproduzierbaren interkonnektierenden Porensystem mit etwa kugelförmigen Poren.

**Beispiel 4**

**Herstellung eines Werkstoffs (Negativ) aus Metall zur Verwendung als Wirbelkörperersatz**

In einem zylindrischen Gefäß werden Kugeln aus geschäumtem Polystyrol (EPS), z.B. Styropor® F 414, mit einem Durchmesser von etwa 1000 µm aufgeschüttet und gerüttelt. Mittels einer aufgesetzten Spritze wird das freie Volumen zwischen den Kugeln unter Druckbeaufschlagung mit einer Keramikmasse gefüllt. Anschließend wird der Rohling im Kühlschrank über 30 Minuten ausgehärtet, dann im Acetonbad von den Polystyrolkugeln befreit und über Aceton getrocknet. Anschließend wird der verbleibende Keramikkörper (Positiv) im Keramikofen mit einer Aufheizrate von 1°C/min über ca. 24 Stunden bis 1300°C gesintert. Danach wird der Ofen über weitere 24 Stunden abgekühlt und das Keramik-Positiv entnommen und anschließend im Muffelofen bei 1000°C im Schleudergußverfahren mit einer CoCrMo-Legierung aufgefüllt. Nach Herausnahme des entstehenden Keramik-Metall-Verbundkörpers wird dieser sandgestrahlt und anschließend im Säurebad von der Keramik befreit. Die erhaltene Metallstruktur (Negativ) stellt einen Kugelverbund dar, wobei die Kugeln über definiert breite Brücken miteinander verbunden sind und ein durchgehend dreidimensionales Fachwerk aus Kugeln bilden. Diese Struktur ist für die Kraftübertragung und das Einwachsen von Knochentrabekeln in ihrer physiologischen Morphologie besonders günstig zu beurteilen. Ein derartiges Implantat ist auch hochstabil und kann direkt als Wirbelkörperersatz eingesetzt werden.

Bei den vorstehenden Beispielen kann statt der genannten Keramikmasse beispielsweise auch Gips verwendet werden, falls die Masse später wieder herausgelöst wird und nicht einen Teil des fertigen Werkstoffes darstellt, also beispielsweise in den Beispielen 3 und 4. Die Verwendung von Gips ist besonders preisgünstig und unproblematisch, da Gips schnell abbindet und einfach verarbeitbar ist. Gips ist üblicherweise bei Temperaturen bis etwa 1050°C beständig und kann deshalb in Kombination mit Metallen oder Metall-Legierungen verwendet werden, deren Schmelztemperatur höchstens 1050°C beträgt.

**Beispiel 5**

**Mit Streben oder Trajektorien verstärktes Implantat**

Beim Wirbelkörperersatz oder zur Verblockung von zwei Wirbelkörpern nach einer Bandscheibenexcision werden Knochenwürfel oder -quader eingesetzt, die sehr hohe Druckkräfte aufnehmen müssen. Bei derartigen Operationen bleiben Reste der Wirbelkörper in unterschiedlicher Weise erhalten. Sind die corticalen Elemente noch erhalten, ist ein Implantat bevorzugt, welches möglichst vom Rande her von allen Seiten durchwachsen werden kann. In einem solchen Falle muß ein Knochenersatzwerkstoff-Implantat ohne corticale Anteile hergestellt werden. Um diesem die nötige Druckfestigkeit zu verleihen, damit es die genannten hohen Beanspruchungen aushält, wird das Implantat mit massiven Streben in axialer Richtung verstärkt.

**Patentansprüche**

1. Verfahren zur Herstellung eines Werkstoffes, mit den folgenden Verfahrensschritten:

   (a) Einfüllen von Formkörpern in eine Form zur Ausbildung einer Schüttung der Formkörper,
   (b) Befüllen der Form mit einem giess-, spritz- oder schüttfähigen Material, wobei die Formkörper einer Druckbeaufschlagung ausgesetzt werden, und Verfestigen des Materials,
   (c) Entfernen der Formkörper, wobei das Material ein Gerüst aus schalenartigen Strukturen mit einem interkonnektierenden Porensystem bildet, dadurch gekennzeichnet, dass die Formkörper deformierbar sind, und dass durch die Einstellung der Druckbeaufschlagung die Konfiguration des fertigen Werkstoffs einstellbar ist.

2. Verfahren nach Anspruch 1 mit dem weiteren Verfahrensschritt:

   (d) Ausgiessen des Gerüstes mit einem giess-, spritz- oder schüttfähigen Material, und Verfestigen des Materials, so dass ein Gerüst aus schalenartigen Strukturen mit einem interkonnektierenden System aus miteinander über Stege verbundenen Formkörpern gebildet wird.

3. Verfahren nach Anspruch 2 mit dem weiteren Verfahrensschritt:

   (e) Entfernen des Gerüstmaterials, so dass nur ein Negativmodell des Gerüsts zurückbleibt, das aus miteinander über Stege verbundenen Formkörpern besteht.

4. Verfahren nach Anspruch 3 mit dem weiteren Verfahrensschritt:

   (f) Ausgiessen des Negativmodells mit einem giess-, spritz- oder schüttfähigen Material, und Verfestigen des Materials, so dass ein Gerüst aus schalenartigen Strukturen mit einem interkonnektierenden System aus miteinander über Stege verbundenen Formkörpern gebildet wird.

**5.** Verfahren nach Anspruch 4 mit dem weiteren Verfahrensschritt:

> (g) Entfernen des Materials des Negativmodells, so dass ein Gerüst aus schalenartigen Strukturen mit einem interkonnektierenden Porensystem gebildet wird.

**6.** Verfahren nach einem der Ansprüche 1-5, wobei im Verfahrensschritt (a) die Form nur soweit mit den deformierbaren Formkörpern gefüllt wird, dass diese dabei nicht wesentlich deformiert werden.

**Claims**

**1.** A method of producing a material, wherein the method comprises the following steps:

> (a) filling deformable moulded articles in a mould for the creation of a bulk of said moulded articles;
> (b) filling said mould with a pourable, extrudable or dumpable material, said moulded articles being pressurized, and wherein said material being hardened;
> (c) removing said moulded articles, wherein said material building a frame of cup-like structures including an interconnecting pore system, characterized in that said molded articles being deformable, and in that the configuration of the finished material can be adjusted by adjusting said pressurization.

**2.** The method according to claim 1, including the further step of:

> (d) pouring said frame with a pourable, extrudable or dumpable material and hardening said material, such that a frame of cup-like structures including a interconnecting system of moulded articles connected through webs will be built.

**3.** The method according to claim 2, including the further step of:

> (e) removing said frame material, so that only one negative model will remain, comprising webs connected to one another.

**4.** The method according to claim 3, including the further step of:

> (f) pouring said negative model with a pourable, extrudable or dumpable material and hardening said material, such that a frame of cup-like structures including a interconnecting system of moulded articles connected through webs will be built.

**5.** The method according to claim 4, including the further step of:

> (g) removing the material of said negative model, so that a frame of cup-like structures including a connected pore system will remain.

**6.** The method according to claims 1 to 4, wherein said mould will only be filled with said deformable molded articles so that the latter will not be significantly deformed.

**Revendications**

**1.** Procédé de fabrication d'une substance selon les étapes suivantes:

> (a) Remplissage d'un moule avec des substances modelables et déformables pour réaliser une masse de ces substances,

> (b) Remplissage du moule avec de la matière pouvant être coulée, injectée ou déversée, ces substances modelables étant soumises à une pression et à une solidification de la matière,

> (c) Enlèvement des substances modelables et déformables, la matière constituant une charpente faite de structures à type de coffrage comportant un système de pores interconnectés, caractérisée par le fait que les substances modelables sont déformables et que de ce fait le réglage de la pression appliquée permet de régler la configuration de la matière terminée.

**2.** Procédé selon revendication 1 avec l'étape suivante:

> (d) Comblement de la charpente à l'aide d'une matière pouvant être coulée, injectée ou déversée, et solidification de la matière, si bien qu'une charpente faite de structures à type de coffrage avec un système réalisant des interconnections au moyen de substances modelables reliées entre elles par des passerelles se trouve constituée.

**3.** Procédé selon revendication 2 avec l'étape suivante:

> (e) Enlèvement de la matière de la charpente, si bien que seul le modèle négatif de la charpente subsiste, constitué par des substances modelables reliées entre elles par des passerelles.

**4.** Procédé selon revendication 3 avec l'étape suivante:

> (f) Comblement du modèle négatif à l'aide d'une matière pouvant être coulée, injectée ou déversée et solidification de la matière, si bien qu'une charpente faite de structures à type de coffrage avec un système réalisant des interconnections au moyen de substances modelables reliées entre elles par des passerelles se trouve constitué.

**5.** Procédé selon revendication 4 avec l'étape suivante:

> (g) Enlèvement de la matière du modèle négatif, si bien que la charpente faite de structures à type de coffrage avec un système de pores réalisant des interconnexions se trouve constituée.

**6.** Procédé selon revendications 1 à 5, le moule n'étant rempli de substances modelables et déformables dans l'étape (a) que jusqu'à ce que celles-ci ne soient pas sensiblement déformées.

Fig. 1

Fig. 2

Fig. 3

Fig. 4

Fig. 5

Fig. 6

Fig. 8

Fig. 7

Fig. 10

Fig. 9

Fig. 11

Fig. 12